Europäisches Patentamt

European Patent Office

Office européen des brevets

(11) Publication number: **0 190 382**
**A1**

(12) **EUROPEAN PATENT APPLICATION**

(21) Application number: **85101215.3**

(22) Date of filing: **06.02.85**

(51) Int. Cl.⁴: **A 61 M 5/14**
**F 16 K 7/06**

(43) Date of publication of application:
**13.08.86 Bulletin 86/33**

(84) Designated Contracting States:
**AT BE CH DE FR GB IT LI LU NL SE**

(71) Applicant: **Hu, Liang-Tung**
**3rd Fl., No. 248, Sec. 4 Hsing-Yi Road**
**Taipei(TW)**

(71) Applicant: **Ueda, Shiro**
**4th Fl. No. 156, Fu Hsing North Road**
**Taipei(TW)**

(72) Inventor: **Hu, Liang-Tung**
**3rd Fl., No. 248 Sec. 4 Hsing Yi Road**
**Taipei(TW)**

(74) Representative: **LOUIS, PÖHLAU, LOHRENTZ & SEGETH**
**Kesslerplatz 1 P.B. 3055**
**D-8500 Nürnberg(DE)**

(54) **Liquid flow controller.**

(57) A liquid flow controller (10) for intravenous injection comprises a first cylindrical member (1) provided at one end surface (15) with a pair of threaded tongues (11, 12) which extend outwardly therefrom and form a recess (13) thereof, a flange (14) being properly disposed on said end surface (15), a second cylindrical member (2) being hollowed and capable of threadedly connected to said first cylindrical member (1), an annular member (3) provided at its first end surface (31) with a hole (311) being received within and secured to said second cylindrical member (2), a controlling rod (4) capable of being received in said annual member (3) with its end (42) passing through said hole (311) thereof, a rubber hose (5) partially surrounded by a rubber ring (6) being positioned in said recess (13) and clamped between said flange (14) and said controlling rod (4), a solid rubber rod (8) capable of being inserted into and received within said rubber hose (5), and a pair of connectors (9) capable of being disposed on both ends of said rubber hose (5) for a better connection.

FIG.1

TITLE: LIQUID FLOW CONTROLLER

The present invention relates to a liquid flow controller which is suitable for an intravenous injection.

It is readily understood that the intravenous injection is an important medical method for patient who is in a rather weak physical condition.

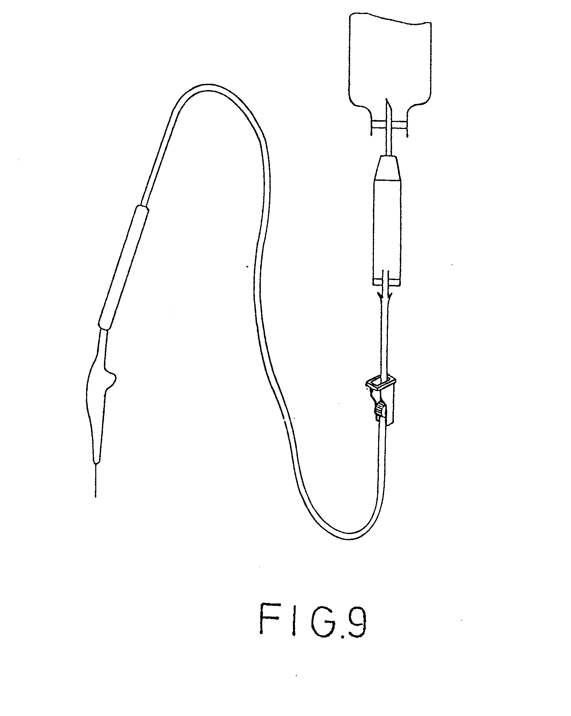

Commonly used apparatus for the intravenous injection, as shown in FIG. 9, mainly comprises a liquid bottle, a plug disposed on the outlet of said liquid bottle, a long P.V.C tube, a pair of injectors and an adjustable flow controller. In use, a first injector passes through said plug and into said liquid bottle which is filled with desired liquid, and one end of said P.V.C tube is connected to said first injector and the other end thereof is connected to a second injector which can be connected to the vein of the patient, and a liquid flow controller can properly disposed on said P.V.C tube to provide a liquid flow control therein.

It is noted that the liquid flow should be properly controlled to correspond to the physical condition of the patient and hence a reliable liquid flow controller is needed.

However, the controlling range of said conventional controller is somewhat limited and hence it is not suitable for the patient who is extremely weak and needs a slower rate intravenous injection. Besides, according to the conventional way, the P.V.C tube is directly pressed by the controller to control the liquid flow therein and hence said P.V.C tube will easily become deformation and therefore it is difficult to obtain a desired liquid flow by means of the conventional apparatus.

It is, therefore, an object of the present invention to provide a liquid flow controller for obtaining a desired liquid flow.

It is another object of the present invention to provide a liquid flow controller, wherein a rubber hose is partially surrounded by a rubber ring for preventing said rubber hose from being directly pressed by the controller during the adjusting period.

It is still another object of the present invention to provide a liquid flow controller, wherein a solid rubber rod is inserted into and received within said rubber hose to provide a desired and accurate liquid flow.

BRIEF DESCRIPTION OF THE DRAWINGS

FIG. 1 is a perspective fragmental view of a preferred embodiment of the present invention;

FIG. 2 is a local perspective fragmental view of the present invention;

FIG. 3 is a perspective view of a liquid flow controller according to the present invention, wherein the rubber hose is positioned in a recess fromed in a first cylindrical member thereof;

FIG. 4 is a perspective view of a liquid flow controller according to the present invention, wherein the rubber hose is clamped between a first and second cylindrical members thereof;

FIG. 5 is a cross-sectional view taken along line 1-1 of FIG. 4;

FIG. 6 shows a P.V.C tube of the prior art which is in a conventional pressed state;

FIG. 7A shows a local cross-sectional view taken along line 2-2 of FIG. 4;

FIG. 7B shows a rubber hose as shown in FIG. 7A which is in a first pressed state;

FIG. 7C shows a rubber hose as shown in FIG. 7A which is in a second pressed state;

FIG. 8 shows a perspective view of the present invention which is cooperated with the known apparatus which are necessary for inravenous injection;

FIG. 9 shows the commonly used apparatus for the intravenous injection;

FIG. 10 shows the different rates of liuqid flow between the present invention and the prior art.

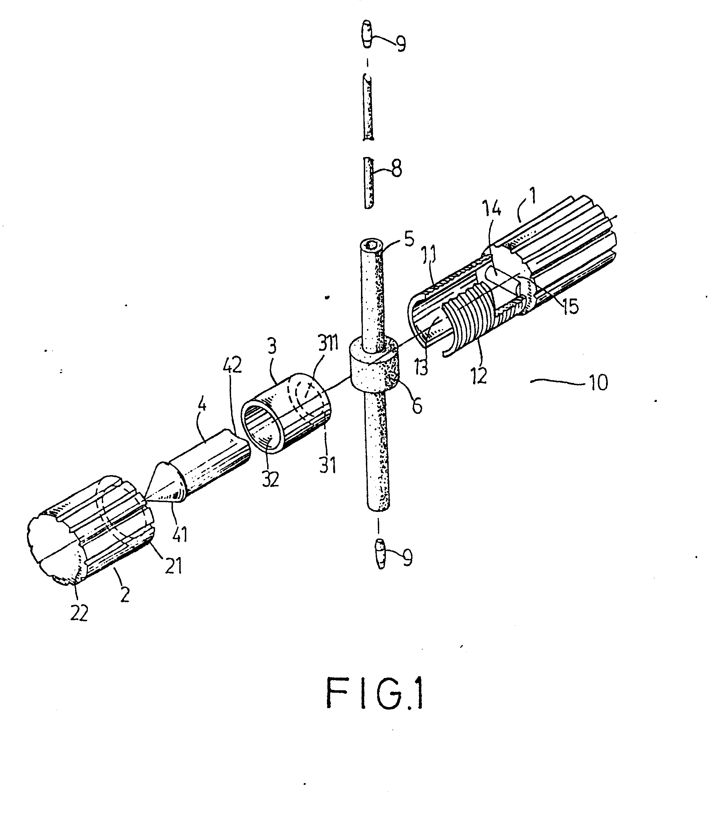

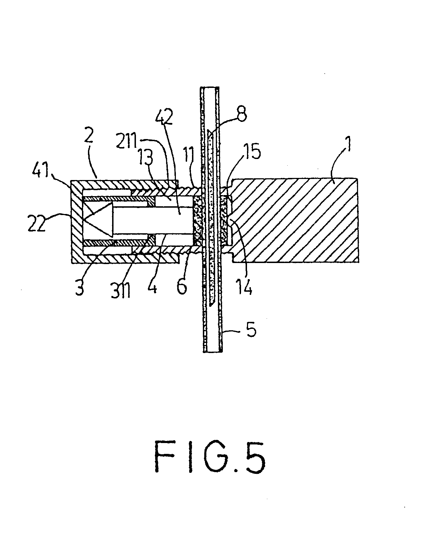

Referring to the drawings and particularly to FIG. 1 a liquid flow controller (10) according to a preferred embodiment of the present invention comprises a first

- 4 -

0190382

cylindrical member (1) provided with a pair of threaded tongues (11) (12) which extend outwardly from one end surface (15) of said first cylindrical member (1) to form a recess (13), a flange (14) properly disposed on said end surface (15), a second cylindrical member (2) being hollowed and provided at a first end (21) with a threaded inner wall (211) (Shown in FIG. 5) and at the other end with a closed second end face (22), an annular member (3) provided at a first end surface (31) with a hole (311) and at the other end with a apertured second end surface (32), a controlling rod (4) provided at one terminal with an enlarged end (41) and at the other terminal with a concaved end (42), said controlling rod (4) capable of being inserted through said apertured second end surface (32) of said annular member (3) and received therewithin in such a manner that said controlling rod (4) may be arranged to move back and forth therein with its concaved end (42) passing through said hole (311) of said annular member (3) as shown in FIG. 5, and with its enlarged end (41) remaining in said annular member (3) to prevent said controlling rod (4) from sliding out of said hole (311) thereof. Said annular member (3) with said controlling rod (4) received therewithin may be inserted through said first end (21) of said second cylindrical member (2) and received therein with its apertured second end face (32) tightly secured to said second end face (22) of said second cylindrical emmber (2) by means of gluing or other suitable ways. It is noted that the enlarged end (41) of said controlling rod (4) contacts said second end face (22) of said second cylindrical emmber (2) only at one single point, hence it may reduce the friction therebetween to make said controlling rod (4) normally operated.

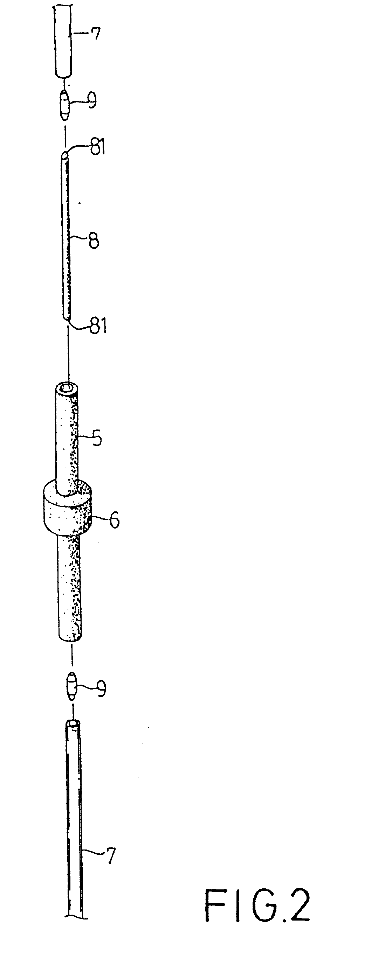

Referring to FIG. 2, a rubber hose (5) connected at both ends to the commonly used P.V.C tubes (7) is partially surrounded by a rubber ring (6) to prevent said rubber hose (5) from being directly pressed by said controlling rod (4) and a solid rubber rod (8) provided at the both ends with shaved portions (81) can be inserted into and received within said rubber hose (5) to form a narrower passage for providing an accurate liquid flow. Said rubber ring (6), rubber hose (5) and solid rubber rod (8) are preferably made of medical grade rubber. A pair of connectors (9) can be disposed on the both ends of said rubber hose (5) to provide a better connection. It is noted that the shaved portions (81) of said solid rubber rod (8) are used to prevent the liquid flow in the rubber hose (5) from being stopped when said solid rubber rod (8) contacts said connectors (9).

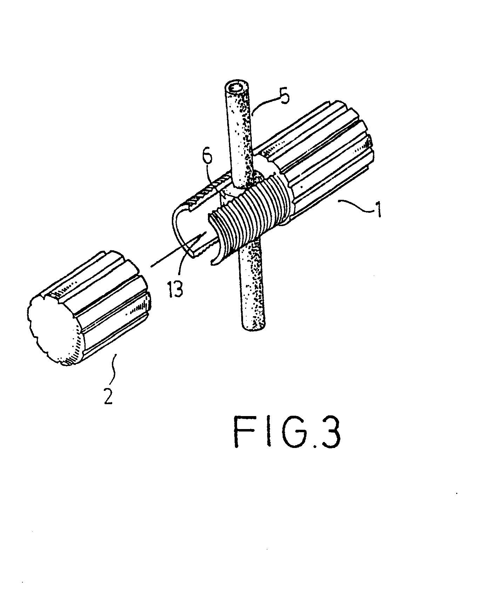

Referring to FIG. 3 the rubber hose (5) together with the rubber ring (6) may be properly positioned in the recess (13) of said first cylindrical member (1).

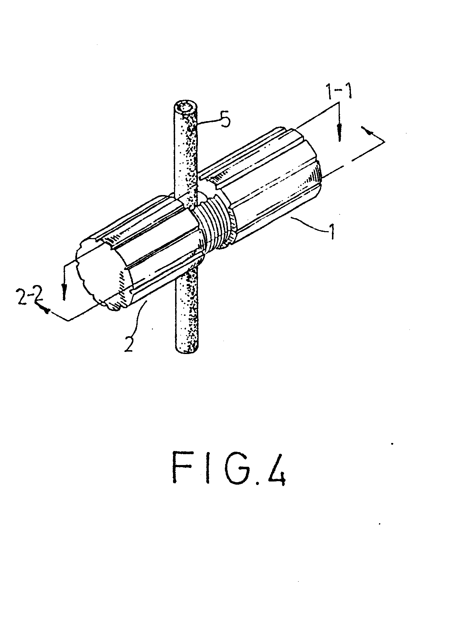

In use, referring to FIGS. 4 and 5, the second cylindrical member (2) is adjustably connected to the first cylindrical member (1) by way of the threaded engagement between said threaded inner wall (211) and said threaded tongues (11) and (12) (as shown in FIG. 5), and the rubber hose (5) together with the rubber ring (6) can be clamped between said controlling rod (4) of said second cylindrical member (2) and said flange (14) of said first cylindrical member (1). When a person properly rotate said second cylindrical member (2) and make it move towards said first cylindrical member (1), the enlarged end (41) of said controlling rod (4) will be pressed by the second end face (22) of said second cylindrical member (2) and the rubber ring (6) together with the rubber hose (5) will in turn be pressed by the concaved end (42) of said controlling rod (4) to provide a desired liquid flow in said rubber hose (5).

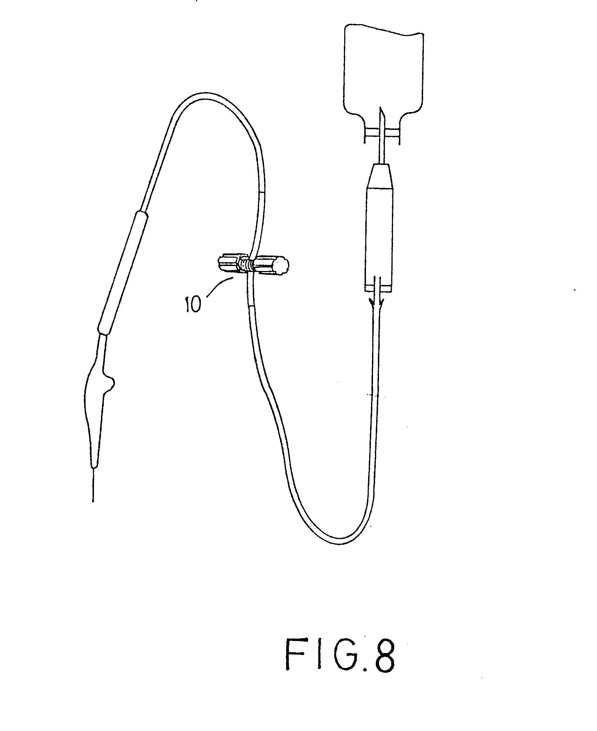

The liquid flow controller (10) according to the present invention cooperated with needed apparatus is shown in FIG. 8.

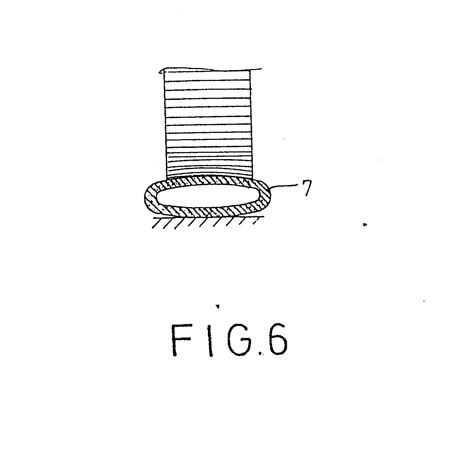

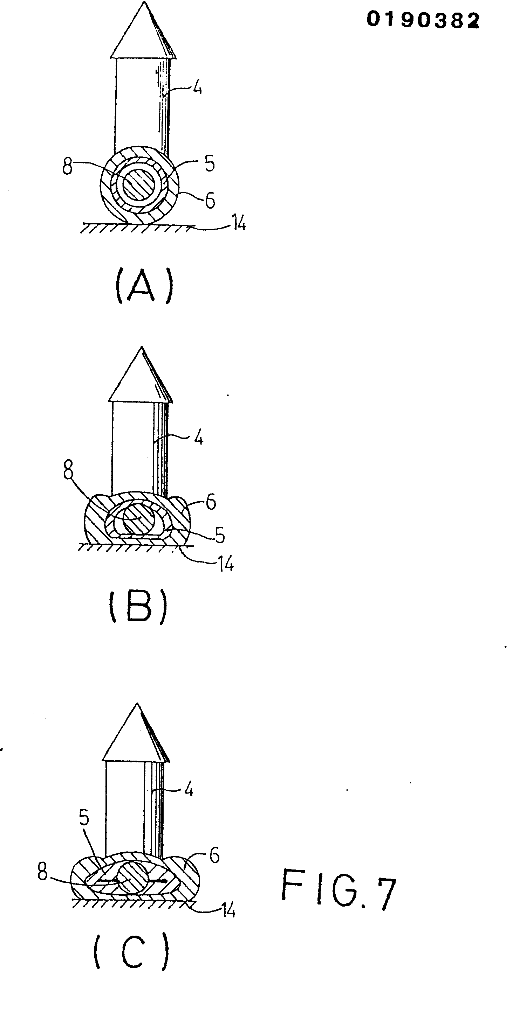

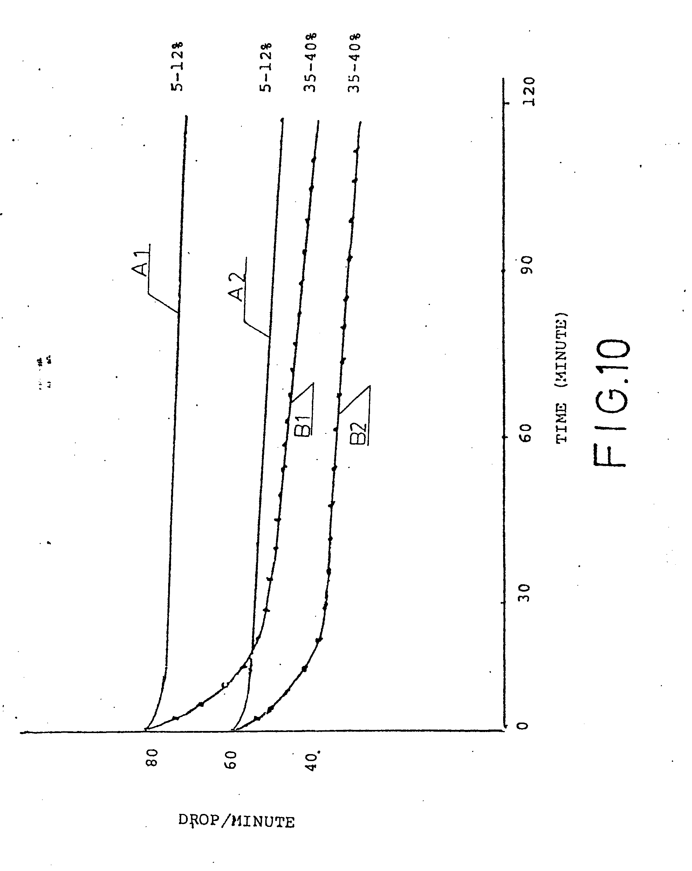

For illustration, FIG. 6 shows a P.V.C tube (7) of the prior art which is in a conventional pressed state, while FIG. 7A shows a rubber hose (5) of the present invention which is properly clamped between said controlling rod (4) and said flange (14), and FIG. 7B shows said rubber hose (5) being in a first pressed state which may allow a desired liquid to pass therethrough, and FIG. 7C shows said rubber hose (5) being in a second pressed state which may prevent the liquid from passing therethrough. For fruther illustration, FIG. 10 shows the different rates of liquid flow between the present invention and the prior art, wherein A1 and A2 respectively represent the present invention with different initial conditions while B1 and B2 respectively represent the prior art of which the initial conditions are respectively similar to those of the present invention. It is seen that the flow rates of A1 and A2, as shown in FIG. 10, are more stable than those of B1 and B2, therefore the present invention can provide a desired and accurate liquid flow in contrast to the prior art and hence the present invention is indeed a better liquid flow controller for intravenous injection.

CLAIMS

1. A liquid flow controller comprising:

a first cylindrical member provided at one end surface with a pair of threaded means which extend outwardly therefrom and form a recess thereof;

a flange properly disposed on said end surface of said first cylindrical member;

a second cylindrical member being hollowed and provided at a first end with a threaded inner wall and at the other end with a closed second end face, said second cylindrical member capable of being threadedly connected to said first cylindrical member;

an annular member provided at a first end surface with a hole and at the other end with a apertured second end surface, said annular member capable of being received within and secured to said second cylindrical member;

a controlling rod provided at one terminal with an enlarged end and at the other terminal with a concaved end, said controlling rod capable of being inserted through said apertured second end surface of said annular member with its concaved end passing through said hole of said first end surface of said annular member and received within said annular member;

characterized in that

a rubber hose through which liquid may pass is partially surrounded by a rubber ring and can be positioned in said recess of said first cylindrical member and clamped between said concaved end of said controlling rod and said flange of said first cylindrical member to control the liquid flow in said rubber hose and a solid rubber rod can be inserted into and received within said rubber hose to form a narrower passage for providing a accurate liquid flow and a pair of connectors can be disposed on the both ends of said rubber hose to provide a better connection.

FIG.1

0190382

FIG.2

FIG.3

# FIG.4

FIG.5

0190382

FIG.6

FIG.7

(A)

(B)

(C)

7/10

0190382

8/10

10

FIG.8

FIG.9

FIG.10

# European Patent Office

## EUROPEAN SEARCH REPORT

EP  85 10 1215

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-2 314 767  (G.H. BURRELL) * page 1, column 1, lines 26-54; figures 1,2 * | 1 | A 61 M   5/14 F 16 K   7/06 |
| | --- | | |
| A | DE-B-1 223 498  (V. LANG) * claim; figures 1-3 * | 1 | |
| | --- | | |
| A | FR-A-1 410 342  (J.-J. ROSIER) * résumé; figures 1-7 * | 1 | |
| | --- | | |
| A | US-A-3 167 085  (W.W. REDMER) * figures 1,3,4 * | 1 | |
| | --- | | |
| A | US-A-3 332 439  (G.K. BURKE) * figures 3,4 * | 1 | |
| | ----- | | |

TECHNICAL FIELDS SEARCHED (Int. Cl.4)

A 61 M   5/00
F 16 K   7/00

The present search report has been drawn up for all claims

| Place of search | Date of completion of the search | Examiner |
|---|---|---|
| BERLIN | 05-09-1985 | MASSALSKI W. |